# FASCICULE DE BREVET EUROPEEN

(11) **EP 1 064 921 B1**
(45) Date de publication et mention de la délivrance du brevet: **03.09.2003**
(21) Numéro de dépôt: 00401595.4
(22) Date de dépôt: 06.06.2000
(51) Int. Cl.: A61K 7/09

(54) **Procédé de permanente comprenant l'application préliminaire d'une composition comprenant au moins un polymère anionique**
Verfahren zum Dauerwellen mit einer Vorbehandlung mit einer Zusammensetzung enthaltend mindestens ein anionisches Polymer
Method for perming with a pretreatment with a composition containing at least an anionic polymer

(30) Priorité: 28.06.1999 FR 9908245
(43) Date de publication de la demande: 03.01.2001
(73) Titulaire: L'OREAL, 75008 Paris (FR)
(72) Inventeur: N'Guyen, Ly-Lan, 94240 L'Hay-Les-Roses (FR); Sabbagh, Anne, 92500 Rueil-Malmaison (FR)
(74) Mandataire: Bourdeau, Françoise

(56) Documents cités:
- DE-A- 19 750 520
- FR-A- 2 739 279
- US-A- 4 240 450
- US-A- 4 660 580

## Description

L'invention a pour objet un procédé de déformation permanente des cheveux comprenant l'application préliminaire d'une composition comprenant au moins un polymère anionique. Elle vise également l'utilisation d'une telle' composition en permanente avant l'application de la composition réductrice.

Il est connu d'utiliser des polymère cationiques dans les compositions réductrice ou fixatrice pour la déformation permanente des cheveux. De manière générale, on sait que ces polymères améliorent les propriétés cosmétiques des cheveux après le traitement. Par exemple, leur toucher est souvent plus doux et leur aspect plus lisse que celui des cheveux traités par des formulation exemptes de tels polymères.

Toutefois, les polymères cationiques présentent généralement l'inconvénient d'alourdir les cheveux qui deviennent mous et manquent de vigueur. Ceci est naturellement néfaste aussi bien pour l'allure de la coiffure que pour sa tenue dans le temps. En effet, contre le souhait de la personne, les boucles réalisées par la permanente commencent par se déformer puis disparaissent rapidement. Enfin, les polymères cationiques rendent souvent les cheveux difficiles à coiffer.

Il existe donc un besoin de trouver un procédé pour la déformation permanente des cheveux qui ne donne pas lieu aux inconvénients exprimés ci-dessus et qui, en particulier, procure aux cheveux de bonnes propriétés cosmétiques, des déformations, des boucles ou des frisures durables et rende, en outre, les cheveux facile à coiffer.

De manière surprenante et inattendue, la Demanderesse a découvert qu'il était possible de résoudre ces problèmes en appliquant sur les cheveux, de façon préliminaire, une composition séparée et distincte des composition réductrice et fixatrice, comprenant au moins un polymère anionique avant d'appliquer la composition réductrice pour permanente.

L'invention a pour objet un procédé de déformation permanente des cheveux comprenant successivement l'application d'une composition réductrice (a) puis d'une composition fixatrice (b), l'une au moins de ces compositions (a) ou (b) comprenant au moins un polymère cationique, caractérisé par le fait qu'avant l'application de la composition réductrice (a), on applique sur les cheveux une troisième composition séparée (c), distincte des compositions (a) et (b), et comprenant au moins un polymère anionique et on rince les cheveux seulement après avoir appliqué la (ou au moins l'une des) composition(s) comprenant le (ou les) polymère(s) cationique(s).

Un autre objet de l'invention concerne l'utilisation d'une composition (c) comprenant au moins un polymère anionique en permanente avant l'application de la composition réductrice (a).

Selon un premier mode de mise en oeuvre avantageux du procédé selon l'invention, seule la composition réductrice (a) comprend un polymère cationique, et on effectue pas de rinçage entre l'application de la composition réductrice (a) et de la composition fixatrice (b).

Selon un second mode de mise en oeuvre avantageux du procédé selon l'invention, seule la composition fixatrice (b) comprend un polymère cationique. Dans ce cas et conformément à la définition générale du procédé selon l'invention, on ne rince pas les cheveux entre l'application de la composition réductrice (a) et de la composition fixatrice (b).

Selon un troisième mode de mise en oeuvre avantageux du procédé selon l'invention, la composition réductrice (a) et la composition fixatrice (b) comprennent toutes les deux un polymère cationique, et on effectue ou non un rinçage entre l'application de la composition réductrice (a) et de la composition fixatrice (b). Plus préférentiellement, on n'effectue pas de rinçage entre l'application de la composition réductrice (a) et de la composition fixatrice (b).

De manière encore plus générale, au sens de la présente invention, l'expression "polymère cationique" désigne tout polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Une famille de polymères cationiques est celle des polymères cationiques siliconés. Parmi ces polymères, on peut citer :
(a) les polymères siliconés répondant à la formule (IV) suivante :

   R⁶ ₐG⁵₃₋ₐ-Si(OSiG⁶ ₂)ₙ-(OSiG⁷ _{b}R⁷ _{2-b})ₘ-O-SiG⁸ _{3-a'}-R⁸ _{a'} (IV)

   dans laquelle :
   G⁵, G⁶, G⁷ et G⁸, identiques ou différents, désignent un atome d'hydrogène, un groupement phényle, OH, alkyle en C₁-C₁₈, par exemple méthyle, alcényle en C₂-C_{18,} ou alcoxy en C₁-C₁₈
   a, a', identiques ou différents, désignent le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10;
   R⁶, R⁷, R⁸, identiques ou différents, désignent un radical monovalent de formule - C_{q}H_{2q}Oₛ R⁹ₜL dans laquelle q est un nombre de 1 à 8, s et t, identiques ou différents, sont égaux à 0 ou à 1, R⁹ désigne un groupement alkylène éventuellement hydroxylé et L est un groupement aminé éventuellement quaternisé choisi parmi les groupements

      -NR"-CH₂-CH₂-N'(R")₂

      -N(R")₂

      -N^{⊕}(R")₃ A⁻

      -N^{⊕}H(R")₂ A⁻

      -N^{⊕}H₂(R")A⁻

      -N(R")-CH₂-CH₂-N^{⊕}R" H₂A⁻,
   dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
   Des produits correspondant à cette définition sont par exemple les polysiloxanes dénommés dans le dictionnaire CTFA "amodiméthicone" et répondant à la formule (V) suivante : 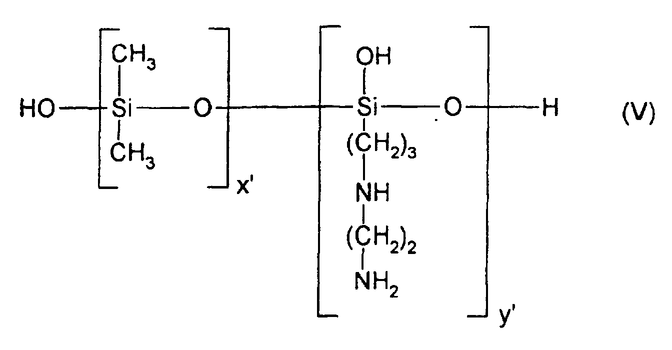 dans laquelle x' et y' sont des nombres entiers dépendant du poids moléculaire, généralement tels que ledit poids moléculaire est compris entre 5 000 et 20 000 environ ;
   Un produit correspondant à la formule (IV) est le polymère dénommé dans le dictionnaire CTFA "triméthylsilylamodiméthicone", répondant à la formule : 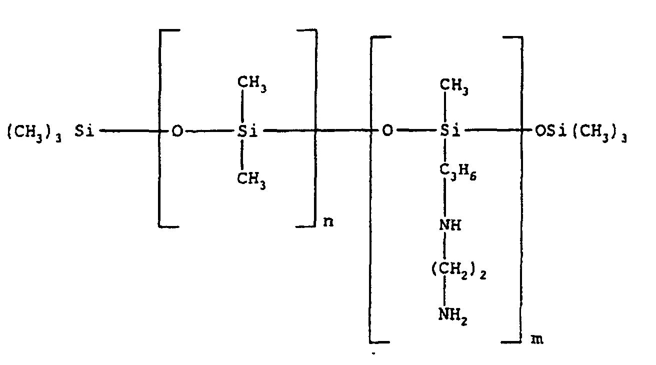 dans laquelle n et m ont les significations données ci-dessus (cf. formule IV).
   Un produit commercial répondant à cette définition est un mélange (90/10 en poids) d'un polydiméthylsiloxane à groupements aminoéthyl aminoisobutyle et d'un polydiméthylsiloxane commercialisé sous la dénomination Q2-8220 par la société DOW CORNING.
   De tels polymères sont décrits par exemple dans la demande de brevet EP-A-95238.
   D'autres polymères répondant à la formule (IV) sont les polymères siliconés répondant à la formule suivante (VI) : 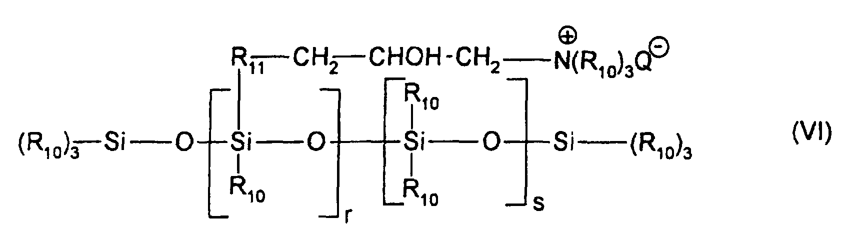 dans laquelle :
   R₁₀ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₁₁ représente un radical hydrocarboné divalent, notamment un radical alkylène en C₁-C₁₈ ou un radical alkylèneoxy divalent en C₁-C₁₈, par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

   De tels polymères sont décrits plus particulièrement dans le brevet US 4 185 087.
b- les composés de formule :

   NH-[(CH₂)₃-Si[OSi(CH₃)₃]]₃

   correspondant à la dénomination CTFA "aminobispropyldiméthicone".

Un polymère entrant dans cette classe est le polymère commercialisé par la Société Union Carbide sous la dénomination "Ucar Silicone ALE 56.

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit commercialisé sous la dénomination "Emulsion Cationique DC 939" par la Société DOW CORNING qui comprend, outre l'amodiméthicone, un agent de surface cationique comprenant un mélange de produits répondant à la formule (VII) : 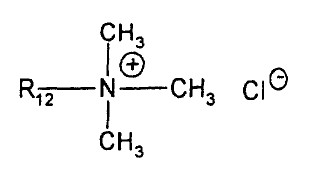 dans lequel R₁₂ désigne des radicaux alcényle et/ou alcoyle ayant de 14 à 22 atomes de carbone, dérivés des acides gras du suif,
en association avec un agent de surface non ionique de formule :

CH₃(CH₂)₁₁-CH₂-(OC₂H₄)ₙ-OH,

N ayant une valeur moyenne de 20.

Un autre produit commercial utilisable selon l'invention est le produit commercialisé sous la dénomination "Dow Coming Q2 7224" par la Société Dow Coming comportant en association le triméthylsilylamodiméthicone de formule (IV), un agent de surface non ionique de formule : C₈H₁₇-C₆H₄-(OCH₂CH₂)ₙ-OH où n = 40 dénommé encore octoxynol-40, un autre agent de surface non ionique de formule : C₁₂H₂₅-(OCH₂-CH₂)ₙ-OH où n = 6 encore dénommé isolaureth-6, et du glycol.

Les polymères du type polyamine, polyamidoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer :
(1) Les copolymères vinylpyrrolidone-acrylate ou -méthacrylate de dialkylamino-alkyle quatemisés ou non, tels que les produits commercialisés sous la dénomination "Gafquat®" par la Société ISP, comme par exemple Gafquat 734, 755 ou HS100 ou bien le produit dénommé "Copolymère 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthyl ammonium, de méthacrylmidopropyl triméthyl ammonium ou de diméthyl-diallylammonium.
   Les produits commercialisés répondant à cette définition sont plus particulièrement les produits commercialisés sous la dénomination "Celquat L 200" et "Celquat H 100" par la Société National Starch.
(4) Les polysaccharides cationiques décrits plus particulièrement dans les brevets US 3 589 578 et 4 031 307 et plus particulièrement le produit commercialisé sous la dénomination "Jaguar C.13 S" commercialisé par la Société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine commercialisés sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5:1 et 1,8:1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclopolymères de méthyl diallyl amine ou de diallyl diméthyl ammonium tels que les homopolymères ou les copolymères comportant comme constituant principal de la chaîne des motifs répondant aux formules (VIII) ou (VIII') : 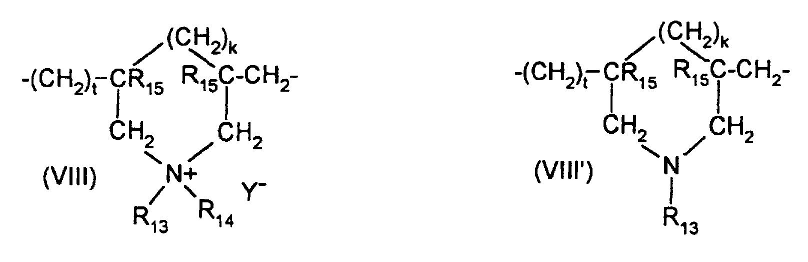 formules dans lesquelles k et t sont égaux à 0 ou 1, la somme k + t étant égale à 1 ; R₁₅ désigne un atome d'hydrogène ou un radical méthyle ; R₁₃ et R₁₄, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₃ et R₁₄ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   On peut citer par exemple l'homopolymère de chlorure de diallyldiméthylammonium commercialisé sous la dénomination "MERQUAT 100" par la société MERCK et les copolymères de chlorure de diallyldiméthylammonium et d'acrylamide commercialisés sous la dénomination "MERQUAT 550".
(10) le polymère de diammonium quaternaire contenant des motifs récurents répondant à la formule (IX) : 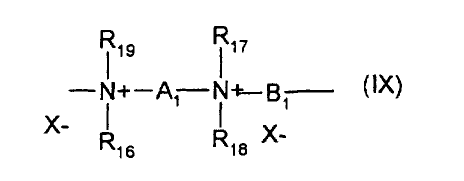 formule (IX) dans laquelle :
   R₁₆, R₁₇, R₁₈ et R₁₉, identiques ou différents, représentent des radicaux aliphatiques, alicycliques, ou arylaliphatiques contenant de 1 à 20 atomes de carbone ou des radicaux hydroxyalkylaliphatiques inférieurs, ou R₁₆, R₁₇, R₁₈ et R₁₉, ensemble ou séparément, constituent avec les atomes d'azote auxquels ils sont rattachés des hétérocycles contenant éventuellement un second hétéroatome autre que l'azote ou bien R₁₆, R₁₇, R₁₈ et R_{19,} représentent un radical alkyle en C₁-C₆ linéaire ou ramifié substitué par un groupement nitrile, ester, acyle, amide ou -CO-O-R₂₀-D ou -CO-NH-R₂₀-D où R₂₀ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₆ et R₁₈ peuvent former avec les deux atomes d'azote auxquels ils sont rattachés un cycle pipérazinique ; en outre si A₁ désigne un radical alkylène ou hydroxyalkylène, linéaire ou ramifié, saturé ou insaturé, B₁ peut également désigner un groupement (CH2)ₙ-CO-D-OC-(CH2)ₙ-
      dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- ;
         De préférence, X⁻ est un anion tel que le chlorure ou le bromure.
         Ces polymères ont une masse moléculaire moyenne en nombre généralement comprise entre 1000 et 100000.
         Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (X): 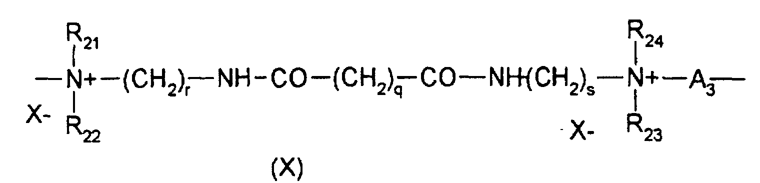 formule dans laquelle :
   R₂₁, R₂₂, R₂₃ et R₂₄, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₂₁, R₂₂, R₂₃ et R₂₄ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A₃ désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol® A-15", "Mirapol® AD1", "Mirapol® AZ1" et "Mirapol® 175" commercialisés par la société Miranol.
(12) les homopolymères ou copolymères dérivés des acides acrylique ou méthacrylique et comportant des motifs : 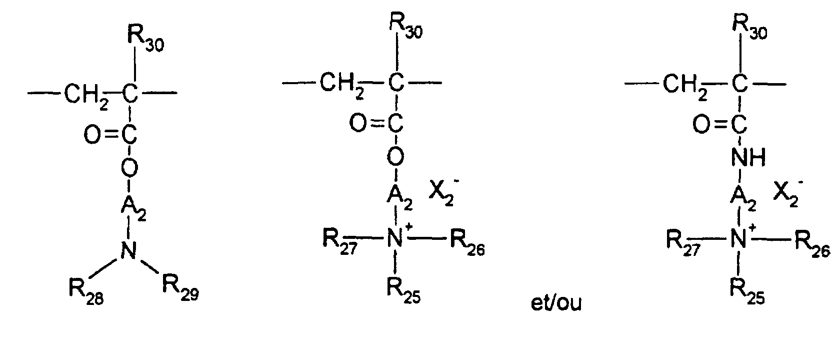 dans lesquels les groupements R₃₀ désignent indépendamment H ou CH₃,
   les groupements A₂ désignent indépendamment un groupe alcoyle linéaire ou ramifié de 1 à 6 atomes de carbone ou un groupe hydroxyalcoyle de 1 à 4 atomes de carbone,
   les groupements R₂₅, R₂₆, R₂₇, identiques ou différents, désignant indépendamment un groupe alcoyle de 1 à 18 atomes de carbone ou un radical benzyle,
   les groupements R₂₈ et R₂₉ représentent un atome d'hydrogène ou un groupement alcoyle de 1 à 6 atomes de carbone,
   X₂⁻ désigne un anion, par exemple méthosulfate ou halogénure, tel que chlorure ou bromure.
      Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations LUVIQUAT® FC 905, FC 550 et FC 370 par la société BASF.
(14) Les polyamines comme le Polyquart H commercialisé par HENKEL, référencé sous le nom de « POLYETHYLENEGLYCOL (15) TALLOW POLYAMINE » dans le dictionnaire CTFA.
(15) Les polymères réticulés de chlorure de méthacryloyloxyéthyl triméthyl ammonium tels que les polymères obtenus par homopolymérisation du diméthylaminoéthylméthacrylate quatemisé par le chlorure de méthyle, ou par copolymérisation de l'acrylamide avec le diméthylaminoéthylméthacrylate quatemisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale. Cette dispersion est commercialisée sous le nom de « SALCARE® SC 95 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères, en particulier les homolymères du chlorure de diméthyldiallylammonium commercialisé sous la dénomination «MERQUAT® 100» par la Société MERCK, les polymères de diammonium quaternaire de formule (IX) ou de formule (X).

Selon l'invention, on peut également utiliser des polymères cationiques sous forme de latex ou de pseudolatex, c'est à dire sous forme d'une dispersion de particules de polymères insolubles.

Selon l'invention, le ou les polymères cationiques peuvent représenter de 0,01% à 20 % en poids, de préférence de 0,1 % à 15 % en poids, et encore plus préférentiellement de 0,5 % à 5 % en poids, du poids total de la composition finale.

Les polymères anioniques généralement utilisés sont des polymères comportant des groupements dérivés d'acide carboxylique, sulfonique ou phosphorique et ont un poids moléculaire moyen en poids compris entre environ 500 et 5.000.000.

1) Les groupements carboxyliques sont apportés par des monomères mono ou diacides carboxyliques insaturés tels que ceux répondant à la formule : 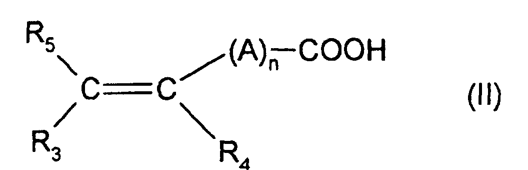 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₅ désigne un atome d'hydrogène, un groupement phényle ou benzyle, R₃ désigne un atome d'hydrogène, un groupement alkyle inférieur ou carboxyle, R₄ désigne un atome d'hydrogène, un groupement alkyle inférieur, un groupement -CH₂-COOH, phényle ou benzyle ;
Dans la formule précitée un radical alkyle inférieur- désigne de préférence un groupement ayant 1 à 4 atomes de carbone et en particulier, méthyle et éthyle.

Les polymères anioniques à groupements carboxyliques préférés selon l'invention sont:
A) les homo- ou copolymères d'acide acrylique ou méthacrylique ou leurs sels et en particulier les produits commercialisés sous les dénominations VERSICOL E ou K par la société ALLIED COLLOID et ULTRAHOLD par la société BASF. Les copolymères d'acide acrylique et d'acrylamide commercialisés sous la forme de leur sel de sodium sous les dénominations RETEN 421, 423 ou 425 par la Société HERCULES, les sels de sodium des acides polyhydroxycarboxyliques.
B) les copolymères des acides acrylique ou méthacrylique avec un monomère monoéthylénique tel que l'éthylène, le styrène, les esters vinyliques, les esters d'acide acrylique ou méthacrylique. Ces copolymères peuvent être greffés sur un polyalkylène glycol tel que le polyéthylène glycol. De tels polymères sont décrits en particulier dans le brevet français 1.222.944 et la demande allemande 2.330.956. On peut notamment citer les copolymères comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide (méth)acrylique et de (méth)acrylate d'alkyle en C₁-C₂₀ par exemple de lauryle (tel que celui commercialisé par la société ISP sous la dénomination ACRYLIDONE LM), de tertiobutyle (LUVIFLEX VBM 70 commercialisé par BASF) ou de méthyle (STEPANHOLD EXTRA commercialisé par STEPAN) et les terpolymères acide méthacrylique/ acrylate d'éthyle/ acrylate de tertiobutyle tel que le produit commercialisé sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 commercialisées par la société NATIONAL STARCH.
D) les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ; De tels polymères sont décrits en particulier dans les brevets US 2.047.398, 2.723.248, 2.102.113, le brevet GB 839.805 et notamment ceux commercialisés sous les dénominations GANTREZ AN ou ES, AVANTAGE CP par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire moyen en poids compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire moyen en poids d'environ 500.000 et d'environ 100.000 commercialisés respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique commercialisé sous la dénomination COSMEDIA POLYMER HSP 1180 par Henkel.

Selon l'invention, les polymères anioniques sont de préférence choisis parmi les copolymères d'acide acrylique tels que les terpolymères acide acrylique / acrylate d'éthyle / N-tertiobutylacrylamide commercialisés notamment sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/ néododécanoate de vinyle commercialisés notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifiés commercialisés par exemple sous la dénomination GANTREZ par la société ISP, les copolymères d'acide méthacrylique et de méthacrylate de méthyle commercialisés sous la dénomination EUDRAGIT L par la société ROHM PHARMA, les copolymères d'acide méthacrylique/ méthacrylate de méthyle / acrylate d'alkyle en C1-C4 / acide acrylique ou méthacrylate d'hydroxyalkyle en C1-C4 commercialisés sous forme de dispersions sous la dénomination AMERHOLD DR 25 par la société AMERCHOL ou sous la dénomination ACUDYNE 255 par la société ROHM & HAAS, les copolymères d'acide méthacrylique et d'acrylate d'éthyle commercialisés sous la dénomination LUVIMER MAEX ou MAE par la société BASF et les copolymères acétate de vinylelacide crotonique, les copolymères acétate de vinylelacide crotonique greffés par du polyéthylèneglycol sous la dénomination ARISTOFLEX A par la société BASF, les homopolymères d'acide acrylique ou méthacrylique commercialisés par exemple sous la dénomination VERSICOL E 5.

Les polymères anioniques les plus particulièrement préférés sont choisis parmi les copolymères méthylvinyléther / anhydride maléique mono estérifiés commercialisés sous la dénomination GANTREZ ES 425 par la société ISP, les terpolymères acide acrylique / acrylate d'éthyle / N-tertiobutylacrylamide commercialisés sous la dénomination ULTRAHOLD STRONG par la société BASF, les copolymères d'acide méthacrylique et de méthacrylate de méthyle commercialisés sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle commercialisés sous la dénomination Résine 28-29-30 par la société NATIONAL STARCH, les copolymères d'acide méthacrylique et d'acrylate d'éthyle commercialisés sous la dénomination LUVIMER MAEX OU MAE par la société BASF, les terpolymères vinylpyrrolidone / acide acrylique / méthacrylate de lauryle commercialisés sous la dénomination ACRYLIDONE LM par la société ISP et les homopolymères d'acide acrylique ou méthacrylique commercialisés par exemple sous la dénomination VERSICOL E 5.

Selon l'invention, on peut également utiliser des polymères anioniques sous forme de latex ou de pseudolatex, c'est à dire sous forme d'une dispersion de particules de polymères insolubles.

Selon l'invention, on peut également utiliser les polymères anioniques de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 1 à 40% en poids d'acide acrylique ;
c) 5 à 40% en poids de macromère siliconé de formule : 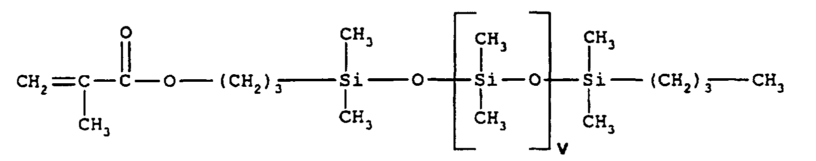 avec v étant un nombre allant de 5 à 700 ; les pourcentages en poids étant calculés par rapport au poids total des monomères.

Une famille de polymères siliconés à squelette polysiloxanique greffé par des monomères organiques non siliconés convenant à la mise en oeuvre de la présente invention est constituée par les polymères siliconés comportant dans leur structure le motif de formule (III) suivant : 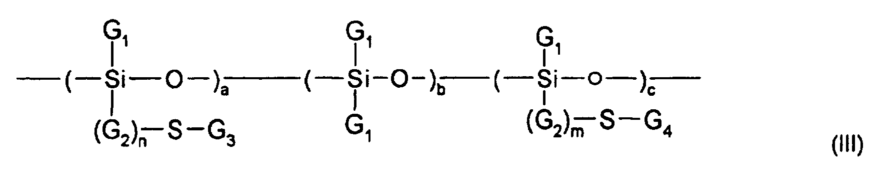 dans lequel les radicaux G₁, identiques ou différents, représentent l'hydrogène ou un radical alkyle en C₁-C₁₀ ou encore un radical phényle ; les radicaux G₂, identiques ou différents, représentent représente un groupe alkylène en C₁-C₁₀; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (III) ci-dessus présente au moins l'une, et encore plus préférentiellement l'ensemble, des caractéristiques suivantes :
- les radicaux G₁ désignent un radical alkyle en C₁-C₁₀ de préférence le radical méthyle
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle en C₁-C₁₀., de préférence le (méth)acrylate d'isobutyle ou de méthyle.

De préférence, le motif de formule (III) ci-dessus peut également représenter l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- c est égal zéro.

Des exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle tel que le poly(méth)acrylate d'isobutyle.
On utilise particulièrement les polymères siliconés greffés de formule (III) de structure polyméthyl/méthylsiloxane à groupements propyl thio-3 acide polyméthacrylique et groupements propyl thio-3 polyméthacrylate de méthyle et les polymères siliconés greffés de formule (III) de structure polyméthyl/méthylsiloxane à groupements propyl thio-3 acide polyacrylique.

De préférence, la troisième composition (c) appliquée avant la composition réductrice (a) comprend entre 0,01 et 20 % de polymère anionique, plus préférentiellement entre 0,1 et 10 %, et plus préférentiellement encore entre 0,2 et 5 %.

Conformément au procédé selon l'invention, on peut utiliser toutes sortes de compositions réductrice (a) et fixatrice (b), et par exemple, celles décrites dans les brevets européens EP 465 342, EP 440 547, EP 568 695, EP 636 358, EP 681 828 ou EP 723 772 appartenant à la Demanderesse ou dans ses demandes de brevet françaises publiées sous les numéro FR 2 773 071, FR 2 773 072 et FR 2 769 499.

On peut utiliser dans la composition tout agent réducteur connu en soi.

Avantageusement, la composition réductrice (a) comprend au moins un agent réducteur thiolé choisi parmi l'acide thioglycolique, l'acide thiolactique, la cystéine, la cystéamine et le thioglycolate de glycérol.

On peut utiliser dans la composition fixatrice (b) tout agent oxydant connu en soi.

Avantageusement, la composition fixatrice (b) comprend avantageusement au moins un agent oxydant choisi parmi le peroxyde d'hydrogène, le peroxyde d'urée, les bromates de métaux alcalins, les persels tels que les perborates et persulfates.

La composition réductrice (a) comprend en outre avantageusement au moins un agent alcalin, pouvant notamment être choisis parmi l'ammoniaque, la monoéthanolamine, la diéthanolamine, la triéthanolamine, l'isopropanolamine, la propanediamine-1,3, un carbonate de bicarbonate alcalin ou d'ammonium, un carbonate organique tel que le carbonate de guanidine ou encore un hydroxyde alcalin, utilisés seul ou en mélange. La composition réductrice (a) peut être formulée en une ou deux parties.

Les compositions fixatrice (b) ou réductrice (a) contiennent, en outre, de préférence, des additifs choisis parmi les tensioactifs, les silicones, des cires, des épaississants, des agents de gonflement et de pénétration, des alcools gras, des dérivés de lanoline, des céramides, des ingrédients actifs, des agents anti-chute, anti-pelliculaires, des agents de mise en suspension, des agents séquestrants, des agents opacifiants, des colorants, des filtres solaires siliconés ou non, des conservateurs, des parfums.

L'invention pourra être mieux comprise à l'aide de l'exemple non limitatif qui suit et qui constitue un mode de mise en oeuvre avantageux du procédé conforme à l'invention.

Les pourcentages indiqués dans les exemples sont des pourcentages relatifs en poids et M.A. signifie matière active, OE signifie une unité d'oxyéthylène.

### EXEMPLE :

### Exemple 1 :

On réalise les trois compositions décrites ci-après : une composition comprenant un polymère anionique, une composition réductrice (a) et une composition fixatrice (b) pour permanente.

### Composition séparée (c) comprenant un polymère anionique :

- Copolymère anionique acide méthacryliqe / acrylate d'éthyle (50/50) 1% MA en dispersion aqueuse ⁽¹⁾
- Monoéthanolamine qs pH 7
- eau déminéralisée qs 100 g

### Composition réductrice (a) :

Le réducteur est un réducteur en deux parties.
- partie A : poudre
   cystéine 3g
   Farine d'Epicéa 5 g
   Farine de tara 2 g
- partie B : liquide
   - Monoéthanolamine 2.2 g
   - Parfum 0.5 g
   - Alcool oléique oxyéthyléné (20 OE)⁽²⁾ 1 g
   - Acide diéthylène triamine pentacétique sel pentasodique en solution aqueuse à 40% 0,4 g
   - Hexadiméthrine chloride (Chimex) 1,2g M.A.
   - Eau déminéralisée qs 90 g

### Composition fixatrice (b) :

- Eau oxygénée 8 % en volume
- Homopolymère chlorure de diméthyl diallyl 1 g M.A. ammonium en solution aqueuse à 40 % ⁽³⁾
- Cocoylamidopropyl dimethyl hydroxypropyl 1g M.A. sulfobetaine en solution aqueuse à 50% ⁽⁴⁾
- Acide citrique qs pH 3
- Eau déminéralisée qs 100 g

(1) LUVIMER MAE de Basf
(2) BRIJ 98V de Uniqema
(3) MERQUAT 100 de Calgon
(4) REWOTERIC AMCAS de Witco

On applique, sur des cheveux lavés et essorés, la troisième composition séparée (c) comprenant le polymère anionique. Ensuite, sans effectuer de rinçage, on applique immédiatement la composition réductrice (a), après avoir mélangé les parties A et B. On met en forme les cheveux aux doigts ou à l'aide d'un peigne. Après un temps de pose de 15 minutes, toujours sans rincer les cheveux, on applique la composition fixatrice (b). On laisse poser 5 minutes puis on rince les cheveux.

On obtient une mise en forme durable des cheveux. Leur toucher est agréable, cosmétique et corporisé. Ils sont en outre facile à coiffer.

### Exemple 2 :

On prépare la composition réductrice (a) suivante :
- Acide thioglycolique 9 g
- Acide diéthylène triamine pentacétique sel pentasodique en solution aqueuse à 40 % 0,4 g
- Ammoniaque à 20 % de NH₃ qs pH 9
- Eau déminéralisée qsp 100g

On applique, sur les cheveux lavés et essorés, la composition de l'exemple 1 contenant le polymère anionique. Ensuite, sans effectuer de rinçage, on applique immédiatement la composition réductrice (a) de l'exemple 2. Après un temps de pose de 15 minutes, on rince les cheveux et on applique la composition fixatrice (b) de l'exemple 1. On laisse poser 5 minutes, on enlève les bigoudis, puis on rince les cheveux. On obtient des boucles durables et nerveuses. Le toucher des cheveux est agréable. Les cheveux sont faciles à coiffer.

## Revendications

1. Procédé de déformation permanente des cheveux comprenant successivement l'application d'une composition réductrice (a) puis d'une composition fixatrice (b), l'une au moins de ces compositions (a) ou (b) comprenant au moins un polymère cationique, **caractérisé par le fait qu'**avant l'application de la composition réductrice (a), on applique sur les cheveux une troisième composition séparée (c), distincte des compositions (a) et (b), et comprenant au moins un polymère anionique et on rince les cheveux seulement après avoir appliqué la (ou au moins l'une des) composition(s) comprenant le (ou les) polymère(s) cationique(s);
et on n'effectue pas un rinçage entre l'application de la composition réductrice (a) et de la composition fixatrice (b), lorsque seule la composition réductrice (a) comprend un polymère cationique.

2. Procédé selon la revendication 1, **caractérisé par le fait que** seule la composition fixatrice (b) comprend un polymère cationique.

3. Procédé selon la revendication 1, **caractérisé par le fait que** la composition réductrice (a) et la composition fixatrice (b) comprennent toutes les deux un polymère cationique, et on effectue ou non un rinçage entre l'application de la composition réductrice (a) et de la composition fixatrice (b).

4. Procédé selon l'une des revendications précédentes, **caractérisé par le fait que** le polymère anionique de la troisième composition séparée (c) est choisi parmi :
- les polymères comportant des motifs carboxyliques dérivant de monomères mono ou diacides carboxyliques insaturés de formule : 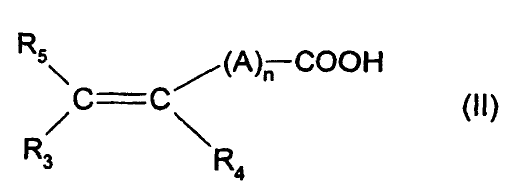
dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₅ désigne un atome d'hydrogène, un groupement phényle ou benzyle, R₃ désigne un atome d'hydrogène, un groupement alkyle inférieur ou carboxyle, R₄ désigne un atome d'hydrogène, un groupement alkyle inférieur, un groupement -CH₂-COOH, phényle ou benzyle.
- les polymères comprenant des motifs dérivant d'acide sulfonique tels que des motifs vinylsulfonique, styrènesulfonique, acrylamido alkylsulfonique.

5. Procédé selon la revendication 4, **caractérisé en ce que** le polymère anionique est choisi parmi :
A) les homo- ou copolymères d'acide acrylique ou méthacrylique ou leurs sels, les copolymères d'acide acrylique et d'acrylamide et leurs sels, les sels de sodium d'acides polyhydroxycarboxyliques.
B) Les copolymères des acides acrylique ou méthacryliques avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol; les copolymères de ce type comportant dans leur chaîne un motif acrylamide éventuellement N-alkylé et/ou hydroxyalkylé, les copolymères d'acide acrylique et de méthacrylate d'alkyle en C₁-C₄ et les terpolymères de vinylpyrrolidone, d'acide acrylique et de méthacrylate d'alkyle en C₁-C₂₀.
C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés.
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées.
E) les polyacrylamides comportant des groupements carboxylates.

6. Procédé selon la revendication 5, **caractérisé en ce que** le polymère anionique est choisi parmi :
- les homopolymères d'acide acrylique ou méthacrylique ;
- les copolymères d'acide acrylique tels que le terpolymère acide acrylique/acrylate d'éthyle/N-tertiobutylacrylamide ;
- les copolymères dérivés d'acide crotonique tels que les terpofymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié.
- les copolymères d'acide méthacrylique et de méthacrylate de méthyle;
- les copolymères d'acide méthacrylique et d'acrylate d'éthyle;
- les terpolymères de vinyipyrrolidone/acide acrylique/méthacrylate de lauryle ;
- les copolymères acétate de vinyle/acide crotonique
- les terpolymères acétate de vinyle/acide crotonique/polyéthylèneglycol.

7. Procédé selon l'une des revendications 1 à 3, **caractérisé par le fait que** le polymère anionique est choisi parmi les polymères anioniques de type siliconés greffés comprenant une portion polysiloxane et une portion constituée d'une chaîne organique non-siliconée, l'une des deux portions constituant la chaîne principale du polymère l'autre étant greffée sur la dite chaîne principale.

8. Procédé selon la revendication 7, **caractérisé par le fait que** le polymère siliconé greffé est choisi parmi les polymères siliconés comportant dans leur structure le motif de formule (III) suivant : 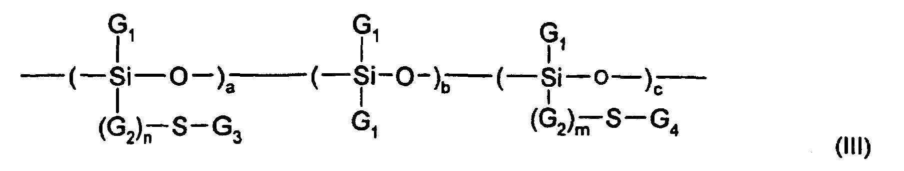 dans lequel les radicaux G₁, identiques ou différents, représentent l'hydrogène ou un radical alkyle en C₁-C₁₀ ou encore un radical phényle ; les radicaux G2, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

9. Procédé selon la revendication 8, **caractérisé par le fait que** le motif de formule (III) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle en C₁-C₁₀ ;

10. Procédé selon la revendication 8 ou 9, **caractérisé par le fait que** le motif de formule (III) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins le (méth)acrylate d'isobutyle ou de méthyle.

11. Procédé selon la revendication 8, **caractérisé par le fait que** le motif de formule (III) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G3 représente un radical polymérique résultant de l'(homo)polymérisation d'au moins un monomère du type acide carboxylique à insaturation éthylénique ;
c est égal à 0.

12. Procédé selon l'une quelconque des revendications précédentes, **caractérisé par le fait que** le polymère cationique est choisi parmi les dérivés d'éther de cellulose quaternaires, les copolymères de cellulose avec un monomère hydrosoluble d'ammonium quaternaire, les cyclopolymères, les polysaccharides cationiques, les polymères cationiques siliconés, les copolymères vinylpyrrolidone-acrylate ou -méthacrylate de dialkylamino-alkyle quatemisés ou non, les polymères quaternaires de vinylpyrrolidone et de vinylimidazole, les polyamidoamines et leurs mélanges.

13. Procédé selon l'une quelconque des revendications 1 à 12, **caractérisé par le fait que** le polymère anionique est utilisé dans la troisième composition séparée (c) en une quantité allant de 0,01 à 20 % en poids du poids total de la composition, de préférence de 0,1 à 10 % en poids et encore plus préférentiellement 0,2 % à 5 % en poids.

14. Procédé selon l'une quelconque des revendications précédentes, **caractérisé par le fait que** la composition réductrice (a) comprend au moins un agent réducteur choisi parmi l'acide thioglycolique, l'acide thiolactique, la cystéine, la cystéamine et le thioglycolate de glycérol.

15. Procédé selon l'une quelconque des revendications précédentes, **caractérisé par le fait que** la composition fixatrice (b) comprend au moins un agent oxydant choisi parmi le peroxyde d'hydrogène, le peroxyde d'urée, les bromates de métaux alcalins, les persels tels que les perborates et persulfates.

16. Procédé selon l'une quelconque des revendications précédentes, **caractérisé par le fait que** la composition réductrice (a) comprend en outre au moins un agent alcalin, pouvant notamment être choisis parmi l'ammoniaque, la monoéthanolamine, la diéthanolamine, la triéthanolamine, l'isopropanolamine, la propanediamine-1,3, un carbonate de bicarbonate alcalin ou d'ammonium, un carbonate organique tel que le carbonate de guanidine ou encore un hydroxyde alcalin, utilisés seul ou en mélange.

17. Procédé selon l'une quelconque des revendications précédentes, **caractérisé par le fait que** la composition réductrice (a) ou fixatrice (b) contient, en outre, des additifs choisis parmi les tensioactifs, les silicones, des cires, des épaississants, des agents de gonflement et de pénétration, des alcools gras, des dérivés de lanoline, des céramides, des ingrédients actifs, des agents anti-chute, anti-pelliculaires, des agents de mise en suspension, des agents séquestrants, des agents opacifiants, des colorants, des filtres solaires siliconés ou non, des conservateurs, des parfums.

## Patentansprüche

1. Verfahren zur dauerhaften Verformung der Haare, das umfasst, eine reduzierende Zusammensetzung (a) und anschließend eine fixierende Zusammensetzung (b) aufzutragen, wobei mindestens eine der Zusammensetzungen (a) oder (b) mindestens ein kationisches Polymer enthält, **dadurch gekennzeichnet, dass** vor dem Aufbringen der reduzierenden Zusammensetzung (a) auf die Haare eine getrennt vorliegende dritte Zusammensetzung (c) aufgetragen wird, die sich von den Zusammensetzungen (a) und (b) unterscheidet und die mindestens ein anionisches Polymer enthält, und die Haare erst gespült werden, nachdem die (oder mindestens eine der) Zusammensetzung(en), die das (oder die) kationische(n) Polymer(e) enthält, aufgebracht wurde und zwischen dem Auftragen der reduzierenden Zusammensetzung (a) und dem Auftragen der fixierenden Zusammensetzung (b) nicht gespült wird, falls nur die reduzierende Zusammensetzung (a) ein kationisches Polymer enthält.

2. Verfahren nach Anspruch 1, **dadurch gekennzeichnet, dass** nur die fixierende Zusammensetzung (b) ein kationisches Polymer enthält.

3. Verfahren nach Anspruch 1, **dadurch gekennzeichnet, dass** die reduzierende Zusammensetzung (a) und die fixierende Zusammensetzung (b) beide ein kationisches Polymer enthalten und zwischen dem Auftragen der reduzierenden Zusammensetzung (a) und dem Auftragen der fixierenden Zusammensetzung (b) gespült oder nicht gespült wird.

4. Verfahren nach einem der vorhergehenden Ansprüche, **dadurch gekennzeichnet, dass** das anionische Polymer der dritten getrennt vorliegenden Zusammensetzung (c) ausgewählt ist unter:
- Polymeren mit Carboxygruppen, die von ungesättigten Mono- oder Dicarbonsäuremonomeren der folgenden Formel abgeleitet sind: 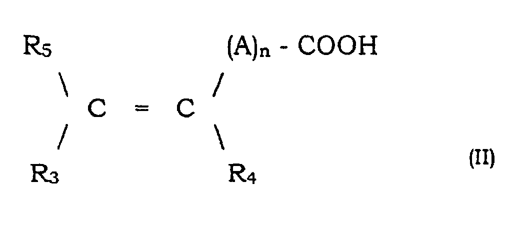
worin bedeuten: n Null oder eine ganze Zahl von 1 bis 10, A eine Methylengruppe, die gegebenenfalls an das Kohlenstoffatom der ungesättigten Gruppe oder, falls n größer 1 ist, an die benachbarte Methylengruppe über ein Heteroatom, wie Sauerstoff oder Schwefel, gebunden ist, R₅ ein Wasserstoffatom, eine Phenylgruppe oder eine Benzylgruppe, R₃ ein Wasserstoffatom, eine niedere Alkylgruppe oder eine Carboxygruppe und R₄ ein Wasserstoffatom, eine niedere Alkylgruppe, eine Gruppe -CH₂-COOH, eine Phenylgruppe oder eine Benzylgruppe; und
- Polymeren, die von einer Sulfonsäure abgeleitete Einheiten enthalten, wie beispielsweise Vinylsulfonsäure-Einheiten, Styrolsulfonsäure-Einheiten oder Acyylamidoalkylsulfonsäure-Einheiten.

5. Verfahren nach Anspruch 4, **dadurch gekennzeichnet, dass** das anionische Polymer ausgewählt ist unter:
A) Homo- oder Copolymeren von Acrylsäure oder Methacrylsäure oder deren Salzen, Copolymeren von Acrylsäure und Acrylamid und deren Salzen und den Natriumsalzen von Polyhydroxycarbonsäuren,
B) Copolymeren von Acrylsäure oder Methacrylsäure mit einem monoethylenisch ungesättigten Monomer, wie Ethylen, Styrol, Vinylestern, Acrylsäure- oder Methacrylsäureestern, die gegebenenfalls auf ein Polyalkylenglykol, wie Polyethylenglykol, gepfropft sind; Copolymeren dieses Typs, die in ihrer Kette eine Acrylamideinheit aufweisen, die gegebenenfalls N-alkyliert und/oder hydroxyalkyliert ist; Copolymeren von Acrylsäure und C₁₋₄-Alkylmethacrylat und Terpolymeren von Vinylpyrrolidon, Acrylsäure und C₁₋₂₀-Alkylmethacrylat,
C) Copolymeren, die von Crotonsäure abgeleitet sind, wie Gopolymeren, die in ihrer Kette Vinylacetat- oder Vinylpropionat-Einheiten und gegebenenfalls weitere Monomere aufweisen, wie Allyiester oder Methallylester, Vinylether oder Vinylester einer gesättigten, geradkettigen oder verzweigten Carbonsäure mit langer Kohlenwasserstoffkette, wie Carbonsäuren mit mindestens 5 Kohlenstoffatomen, wobei diese Polymere gegebenenfalls gepfropft sein können,
D) Polymeren, die von Maleinsäure, Fumarsäure, Itaconsäure oder deren Anhydriden und Vinylestern, Vinylethern, Vinylhalogeniden, Phenylvinylderivaten oder Acrylsäure und ihren Estern abgeleitet sind; Copolymeren von Maleinsäureanhydrid, Citraconsäureanhydrid oder Itaconsäureanhydrid mit einem Allylester oder einem Methallylester, die in ihrer Kette gegebenenfalls eine der folgenden Gruppen enthalten: Acrylamid, Methacrylamid, α-Olefin, Acrylester, Methacrylester, Acrylsäure, Methacrylsäure oder Vinylpyrrolidon, wobei die Anhydridgruppen einfach verestert sind oder als einfaches Amid vorliegen, und
E) Polyacrylamiden mit Carboxylatgruppen.

6. Verfahren nach Anspruch 5, **dadurch gekennzeichnet, dass** das aasionische Polymer ausgewählt ist unter:
- Homopolymeren von Acrylsäure oder Methacrylsäure,
- Copolymeren von Acrylsäure, wie dem Acrylsäure/Ethylacrylat/Nt-Butylacrylamid-Terpolymer,
- Copolymeren, die von Crotonsäure abgeleitet sind, wie Vinylacetat/Vinyl-*t*-butylbenzoat/Crotonsäure-Terpolymeren und Crotonsäure/Vinylacetat/Vinylneododecanoat-Terpolymeren,
- Polymeren, die von Maleinsäure, Fumarsäure, Itaconsäure oder deren Anhydriden und Vinylestern, Vinylethern, Vinylhalogeniden, Phenylvinylderivaten, Acrylsäure und ihren Estern abgeleitet sind, wie den Copolymeren von Methylvinylether und einfach verestertem Maleinsäureanhydrid,
- Copolymeren von Methacrylsäure und Methylmethacrylat,
- Copolymeren von Methacrylsäure und Ethylacrylat,
- Vinylpyrrolidon/Acyylsäure/Lauyylmethacrylat-Terpolymeren,
- Vinylacetat/Crotonsäure-Copolymeren, und
- Vinylacetat/Crotonsäure/Polyethylenglykol-Terpolymeren.

7. Verfahren nach einem der Ansprüche 1 bis 3, **dadurch gekennzeichnet, dass** das anionische Polymer unter den anionischen Polymeren vom Typ der gepfropften Siliconpolymere ausgewählt ist, die einen Polysiloxan-Bestandteil und einen Bestandteil aufweisen, der aus einer nicht siliconhaltigen organischen Kette besteht, wobei ein Bestandteil die Hauptkette des Polymers bildet und der andere Bestandteil auf die Hauptkette gepfropft ist.

8. Verfahren nach Anspruch 7, **dadurch gekennzeichnet, dass** das gepfropfte Siliconpolymer unter den Siliconpolymeren ausgewählt ist, die in ihrer Struktur die Einheit der folgenden Formel (III) aufweisen: 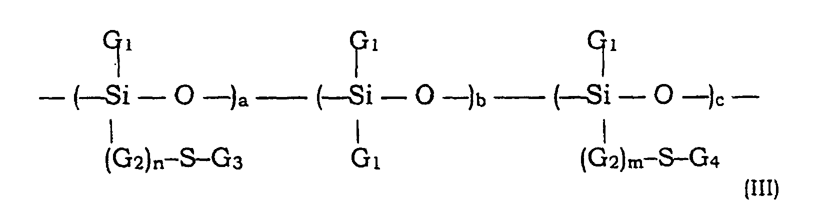 worin bedeuten:
die Gruppen G₁, die identisch oder voneinander verschieden sind, Wasserstoff oder eine Allylgruppe mit 1 bis 10 Kohlenstoffatomen oder auch eine Phenylgruppe, die Gruppen G₂, die identisch oder voneinander verschieden sind, eine Alkylengruppe mit 1 bis 10 Kohlenstoffatomen, G₃ eine Polymergruppe, die das Ergebnis der (Homo)polymerisation mindestens eines anionischen Monomers mit ethylenischer Doppelbindung ist, G₄ eine Polymergruppe, die bei der (Homo)polymerisation mindestens eines hydrophoben Monomers mit ethylenischer Doppelbindung entsteht, m und n Null oder 1, a Null oder eine ganze Zahl von 1 bis 50, b eine ganze Zahl, die im Bereich von 10 bis 350 liegen kann, und c Null oder eine ganze Zahl von 1 bis 50,
mit der Maßgabe, dass einer der Parameter a und c von Null verschieden ist.

9. Verfahren nach Anspruch 8, **dadurch gekennzeichnet, dass** die Einheit der Formel (III) mindestens eine der folgenden Eigenschaften aufweist:
- Die Gruppen G₁ bedeuten eine Alkylgruppe mit 1 bis 10 Kohlenstoffatomen,
- n ist nicht Null und die Gruppen G₂ bedeuten eine zweiwertige Gruppe mit 1 bis 3 Kohlenstoffatomen,
- G₃ bedeutet eine Polymergruppe, die das Ergebnis der (Homo)polymerisation mindestens eines Monomers vom Typ einer Carbonsäure mit ethylenischer Doppelbindung ist, und
- G₄ bedeutet eine Polymergruppe, die bei der (Homo)polymerisation mindestens eines Monomers vom Typ C₁₋₁₀-Alkyl(meth)acrylat gebildet wird.

10. Verfahren nach Anspruch 8 oder 9, **dadurch gekennzeichnet, dass** die Einheit der Formel (III) gleichzeitig sämtliche folgenden Eigenschaften aufweist:
- Die Gruppen G₁ bedeuten eine Methylgruppe,
- n ist nicht Null und die Gruppen G₂ bedeuten eine Propylengruppe,
- G₃ bedeutet eine Polymergruppe, die durch (Homo)polymerisation von mindestens Acrylsäure und/oder Methacrylsäure entsteht, und
- G₄ bedeutet eine Polymergruppe, die das Ergebnis der (Homo)polymerisation von mindestens Isobutyl(meth)acrylat oder Methyl-(meth)acrylat ist.

11. Verfahren nach Anspruch 8, **dadurch gekennzeichnet, dass** die Einheit der Formel (III) mindestens eine der folgenden Eigenschaften aufweist:
- Die Gruppen G₁ bedeuten eine Alkylgruppe mit 1 bis 10 Kohlenstoffatomen,
- n ist nicht Null und die Gruppen G₂ bedeuten eine zweiwertige Gruppe mit 1 bis 3 Kohlenstoffatomen,
- G₃ bedeutet eine Polymergruppe, die das Ergebnis der (Homo)polymerisation mindestens eines Monomers vom Typ einer Carbonsäure mit ethylenischer Doppelbindung ist, und
- c ist 0.

12. Verfahren nach einem der vorhergehenden Ansprüche, **dadurch gekennzeichnet, dass** das kationische Polymer ausgewählt ist unter quartären Celluloseetherderivaten, Copolymeren von Cellulose und einem wasserlöslichen quartären Ammoniummonomer, Cyclopolymeren, kationischen Polysacchariden, kationischen Siliconpolymeren, quaternisierten oder nicht quaternisierten Copolymeren von Vinylpyrrolidon und Dialkylaminoalkylacrylat oder Dialkylaminoalkylmethacrylat, quartären Polymeren von Vinylpyrrolidon und Vinylimidazol, Polyaminoamiden und den Gemischen dieser Verbindungen.

13. Verfahren nach einem der Ansprüche 1 bis 12, **dadurch gekennzeichnet, dass** das in der getrennt vorliegenden drittem Zusammensetzung (c) verwendete anionische Polymer in einem Mengenanteil von 0,01 bis 20 Gew.-%, vorzugsweise von 0,1 bis 10 Gew.-% und noch bevorzugter von 0,2 bis 5 Gew.-% verwendet wird.

14. Verfahren nach einem der vorhergehenden Ansprüche, **dadurch gekennzeichnet, dass** die reduzierende Zusammensetzung (a) ein Reduktionsmittel enthält, das unter Thioglykolsäure, Thiomilchsäure, Cystein, Cysteamin und Glycerinthioglykolat ausgewählt ist.

15. Verfahren nach einem der vorhergehenden Ansprüche, **dadurch gekennzeichnet, dass** die fixierende Zusammensetzung (b) mindestens ein Oxidationsmittel enthält, das unter Wasserstoffperoxid, Harnstoffperoxid, Alkalimetallbromaten und Salzen von Persäuren, wie Perboraten und Persulfaten, ausgewählt ist.

16. Verfahren nach einem der vorhergehenden Ansprüche, **dadurch gekennzeichnet, dass** die reduzierende Zusammensetzung (a) ferner mindestens ein Alkalisierungsmittel enthält, das insbesondere unter Ammoniak; Monoethanolamin, Diethanolamin, Triethanolamin, Isopropanolamin, 1,3-Diaminopropan, Alkalicarbonaten, Alkalihydrogencarbonaten, Ammoniumcarbonat, Ammoniumhydrogencarbonat, organischen Carbonaten, wie Guanidincarbonat, oder Alkalihydroxiden ausgewählt ist, wobei diese Verbindungen einzeln oder im Gemisch verwendet werden.

17. Verfahren nach einem der vorhergehenden Ansprüche, **dadurch gekennzeichnet, dass** die reduzierende Zusammensetzung (a) oder die fixierende Zusammensetzung (b) außerdem Zusatzstoffe enthalten, die unter den grenzflächenaktiven Stoffen, Siliconen, Wachsen, Verdickungsmitteln, Quellmitteln, Penetrationsmitteln, Fettalkoholen, Lanolinderivaten, Ceramiden, Wirkstoffen, Mitteln gegen Haarausfall, Mitteln gegen Schuppen, Suspendiermitteln, Maskierungsmitteln, Trübungsmitteln, Farbmitteln, siliconierten oder nicht siliconierten Filtern, Konservierungsmitteln und Parfums ausgewählt sind.

## Claims

1. Process for the permanent deformation of the hair successively comprising the application of a reducing composition (a) and then of a neutralizing composition (b), at least one of these compositions (a) or (b) comprising at least one cationic polymer, **characterized in that**, before the application of the reducing composition (a), a third separate composition (c), which composition is distinct from the compositions (a) and (b) and comprises at least one anionic polymer, is applied to the hair and the hair is rinsed only after having applied the (or at least one of the) composition(s) comprising the cationic polymer(s);
and rinsing is not carried out between the application of the reducing composition (a) and of the neutralizing composition (b) when only the reducing composition (a) comprises a cationic polymer.

2. Process according to Claim 1, **characterized in that** only the neutralizing composition (b) comprises a cationic polymer.

3. Process according to Claim 1, **characterized in that** the reducing composition (a) and the neutralizing composition (b) both comprise a cationic polymer and rinsing is or is not carried out between the application of the reducing composition (a) and of the neutralizing composition (b).

4. Process according to one of the preceding claims, **characterized in that** the anionic polymer of the third separate composition (c) is chosen from:
- polymers comprising carboxyl units deriving from unsaturated mono- or dicarboxylic acid monomers of formula: 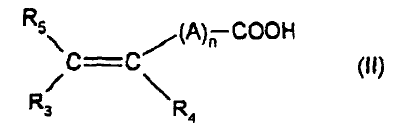 in which n is an integer from 0 to 10, A denotes a methylene group, optionally bonded to the carbon atom of the unsaturated group or to the neighbouring methylene group when n is greater than 1 via a heteroatom, such as oxygen or sulphur, R₅ denotes a hydrogen atom or a phenyl or benzyl group, R₃ denotes a hydrogen atom or a lower alkyl or carboxyl group, and R₄ denotes a hydrogen atom, a lower alkyl group or a -CH₂-COOH, phenyl or benzyl group;
- polymers comprising units deriving from sulphonic acid, such as vinylsulphonic, styrenesulphonic or acrylamidoalkylsulphonic units.

5. Process according to Claim 4, **characterized in that** the anionic polymer is chosen from:
A) homo- or copolymers of acrylic or methacrylic acid or their salts, copolymers of acrylic acid and of acrylamide and their salts, or the sodium salts of polyhydroxycarboxylic acids;
B) copolymers of acrylic or methacrylic acid with a monoethylenic monomer, such as ethylene, styrene, vinyl esters or esters of acrylic or methacrylic acid, optionally grafted onto a polyalkylene glycol, such as polyethylene glycol; the copolymers of this type comprising, in their chain, an optionally N-alkylated and/or hydroxyalkylated acrylamide unit; copolymers of acrylic acid and of C₁-C₄ alkyl methacrylate and terpolymers of vinylpyrrolidone, of acrylic acid and of C₁-C₂₀ alkyl methacrylate;
C) copolymers derived from crotonic acid, such as those comprising, in their chain, vinyl acetate or propionate units and optionally other monomers, such as allyl or methallyl esters, vinyl ether or vinyl ester of a linear or branched saturated carboxylic acid comprising a long hydrocarbonaceous chain, such as those comprising at least 5 carbon atoms, it optionally being possible for these polymers to be grafted;
D) copolymers derived from maleic, fumaric or itaconic acids or anhydrides with vinyl esters, vinyl ethers, vinyl halides, phenylvinyl derivatives, or acrylic acid and its esters; copolymers of maleic, citraconic or itaconic anhydrides and of an allyl or methallyl ester optionally comprising an acrylamide or methacrylamide group, an α-olefin, acrylic or methacrylic esters, acrylic acid, methacrylic acid or vinylpyrrolidone in their chain, ,the anhydride functional groups being monoesterified or monoamidated;
E) polyacrylamides comprising carboxylate groups.

6. Process according to Claim 5, **characterized in that** the anionic polymer is chosen from:
- acrylic or methacrylic acid homopolymers;
- acrylic acid copolymers, such as the acrylic acid/ethyl acrylate/N-tert-butylacrylamide terpolymer;
- copolymers derived from crotonic acid, such as the vinyl acetate/vinyl tert-butylbenzoate/crotonic acid terpolymers and the crotonic acid/vinyl acetate/vinyl neododecanoate terpolymers;
- polymers derived from maleic, fumaric or itaconic acids or anhydrides with vinyl esters, vinyl ethers, vinyl halides, phenylvinyl derivatives, or acrylic acid and its esters, such as the monoesterified methyl vinyl ether/maleic anhydride copolymers;
- copolymers of methacrylic acid and of methyl methacrylate;
- copolymers of methacrylic acid and of ethyl acrylate;
- vinylpyrrolidone/acrylic acid/lauryl methacrylate terpolymers;
- vinyl acetate/crotonic acid copolymers;
- vinyl acetate/crotonic acid/polyethylene glycol terpolymers.

7. Process according to one of Claims 1 to 3, **characterized in that** the anionic polymer is chosen from anionic polymers of grafted silicone type comprising a polysiloxane portion and a portion composed of a non-silicone organic chain, one of the two portions constituting the main chain of the polymer and the other being grafted onto the said main chain.

8. Process according to Claim 7, **characterized in that** the grafted silicone polymer is chosen from silicone polymers comprising, in their structure, the following unit of formula (III): 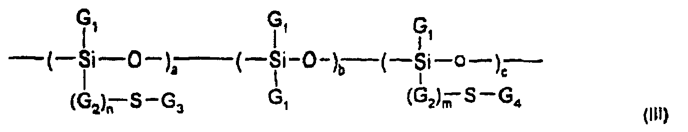 in which the G₁ radicals, which are identical or different, represent hydrogen or a C₁-C₁₀ alkyl radical or a phenyl radical; the G₂ radicals, which are identical or different, represent a C₁-C₁₀ alkylene group; G₃ represents a polymeric residue resulting from the (homo)polymerization of at least one anionic monomer possessing ethylenic unsaturation; G₄ represents a polymeric residue resulting from the (homo)polymerization of at least one hydrophobic monomer possessing ethylenic unsaturation; m and n are equal to 0 or 1; a is an integer ranging from 0 to 50; b is an integer which can be between 10 and 350 and c is an integer ranging from 0 to 50; with the proviso that one of the parameters a and c is other than 0.

9. Process according to Claim 8, **characterized in that** the unit of formula (III) exhibits at least one of the following characteristics:
- the G₁ radicals denote a C₁-C₁₀ alkyl radical;
- n is non-zero and the G₂ radicals represent a divalent C₁-C₃ radical;
- G₃ represents a polymeric radical resulting from the (homo)polymerization of at least one monomer of the carboxylic acid possessing ethylenic unsaturation type;
- G₄ represents a polymeric radical resulting from the (homo)polymerization of at least one monomer of the C₁-C₁₀ alkyl (meth)acrylate type.

10. Process according to Claim 8 or 9, **characterized**
**in that** the unit of formula (III) simultaneously exhibits the following characteristics:
- the G₁ radicals denote a methyl radical;
- n is non-zero and the G₂ radicals represent a propylene radical;
- G3 represents a polymeric radical resulting from the (homo)polymerization of at least acrylic acid and/or methacrylic acid;
- G₄ represents a polymeric radical resulting from the (homo)polymerization of at least isobutyl or methyl (meth)acrylate.

11. Process according to Claim 8, **characterized in that** the unit of formula (III) exhibits at least one of the following characteristics:
- the G₁ radicals denote a C₁-C₁₀ alkyl radical;
- n is non-zero and the G₂ radicals represent a divalent C₁-C₃ radical;
- G₃ represents a polymeric radical resulting from the (homo)polymerization of at least one monomer of the carboxylic acid possessing ethylenic unsaturation type;
- c is equal to 0.

12. Process according to any one of the preceding claims, **characterized in that** the cationic polymer is chosen from quaternary cellulose ether derivatives, copolymers of cellulose with a watersoluble quaternary ammonium monomer, cyclopolymers, cationic polysaccharides, silicone cationic polymers, optionally quaternized vinylpyrrolidone/dialkylaminoalkyl acrylate or methacrylate copolymers, quaternary polymers of vinylpyrrolidone and of vinylimidazole, polyaminoamides and their mixtures.

13. Process according to any one of Claims 1 to 12, **characterized in that** the anionic polymer is used in the third separate composition (c) in an amount ranging from 0.01 to 20% by weight of the total weight of the composition, preferably from 0.1 to 10% by weight and more preferably still 0.2% to 5% by weight.

14. Process according to any one of the preceding claims, **characterized in that** the reducing composition (a) comprises at least one reducing agent chosen from thioglycolic acid, thiolactic acid, cysteine, cysteamine and glyceryl thioglycolate.

15. Process according to any one of the preceding claims, **characterized in that** the neutralizing composition (b) comprises at least one oxidizing agent chosen from hydrogen peroxide, urea hydrogen peroxide, alkali metal bromates, or persalts, such as perborates and persulphates.

16. Process according to any one of the preceding claims, **characterized in that** the reducing composition (a) comprises, in addition, at least one alkaline agent which can be chosen in particular from aqueous ammonia, monoethanolamine, diethanolamine, triethanolamine, isopropanolamine, 1,3-propanediamine, an alkali metal or ammonium carbonate or bicarbonate, an organic carbonate, such as guanidine carbonate, or an alkaline hydroxide, which compounds are used alone or as a mixture.

17. Process according to any one of the preceding claims, **characterized in that** the reducing composition (a) or neutralizing composition (b) comprises, in addition, additives chosen from surfactants, silicones, waxes, thickeners, swelling and penetration agents, fatty alcohols, lanolin derivatives, ceramides, active ingredients, agents for combating hair loss, antidandruff agents, suspending agents, sequestering agents, opacifying agents, colorants, silicone or non-silicone sunscreens, preservatives or fragrances.
